# EUROPÄISCHE PATENTSCHRIFT

(11) **EP 1 189 858 B1**
(45) Veröffentlichungstag und Bekanntmachung des Hinweises auf die Patenterteilung: **22.09.2004**
(21) Anmeldenummer: 00945749.0
(22) Anmeldetag: 14.06.2000
(51) Int. Cl.: C07C 29/145

(54) **VERFAHREN ZUR HERSTELLUNG VON MANNIT**
METHOD FOR PRODUCING MANNITOL
PROCEDE DE PRODUCTION DE MANNITE

(30) Priorität: 25.06.1999 DE 19929368
(43) Veröffentlichungstag der Anmeldung: 27.03.2002
(73) Patentinhaber: MERCK PATENT GmbH, 64293 Darmstadt (DE)
(72) Erfinder: MOHR, Thomas, D-64287 Darmstadt (DE); SCHWARZ, Eugen, D-64625 Bensheim (DE); MACKERT, Peter-Johannes, D-63329 Egelsbach (DE)
(86) Internationale Anmeldenummer: PCT/EP2000/005454
(87) Internationale Veröffentlichungsnummer: WO 2001/000550

(56) Entgegenhaltungen:
- EP-A- 0 006 313
- DE-A- 19 720 496
- CHEMICAL ABSTRACTS, vol. 118, no. 11, 15. März 1993 (1993-03-15) Columbus, Ohio, US; abstract no. 102391a, Seite 898; XP002151789 & HU 60 230 A (MTA, KOZPONTI KEMIAI KUTATO INTEZET) 28. August 1992 (1992-08-28) in der Anmeldung erwähnt
- M. HEGEDÜS, ET AL.: "Stereoselective hydrogenation of D-fructose to D-mannitol on skeletal and supported copper-containing catalysts" STUDIES IN SURFACE SCIENCE AND CATALYSIS, Bd. 78, Heterogeneous Catalysis and Fine Chemicals III, 1993, Seiten 187-194, XP000953460 Elsevier Science, Amsterdam, NL ISSN: 0167-2991
- M. MAKKEE, ET AL.: "Hydrogenation of D-fructose and D-fructose/D-glucose mixtures" CARBOHYDRATE RESEARCH., Bd. 138, Nr. 2, 15. Mai 1985 (1985-05-15), Seiten 225-236, XP002151788 Elsevier Science Publishers, Amsterdam, NL ISSN: 0008-6215 in der Anmeldung erwähnt

## Beschreibung

Die vorliegende Erfindung betrifft ein Verfahren zur Herstellung von Mannit durch Hydrierung von Fructose. Die Hydrierung erfolgt in Gegenwart eines Raney-Kupfer Katalysators und wird in kontinuierlicher Fahrweise durchgeführt.

Mannit findet beispielsweise in der Tablettenproduktion und als Zuckeraustauschstoff Verwendung.

Verfahren zur Herstellung von Mannit aus Fructose durch Hydrierung an heterogenen Kupfer-Katalysatoren sind bereits bekannt.

Geträgerte Kupferkatalysatoren wie z.B. Kupfer auf Kieselgel zeigen in diskontinuierlicher Fahrweise bei der Hydrierung von 10 Gew.-%iger wäßriger Fructose-Lösung Selektivitäten zu Mannit von 60-65% [M. Makkee, A.P.G. Kieboom, H. Bekkum, Carbohydr. Res., 138 (1985) 225].

In der EP 0 006 313 werden geträgerte Kupfer-Katalysatoren für die Fructose-Hydrierung 20 Gew.-%iger wäßriger Fructose-Lösungen verwendet. Der Einsatz eines Kupfer/SiO₂-Katalysators liefert bei der in diskontinuierlicher Fahrweise erfolgenden Fructose-Hydrierung 60-65% an Mannit. Eine Lagerung derartiger Katalysatoren für einige Tage muß unter Wasserstoffatmosphäre erfolgen.

Die in diskontinuierlicher Fahrweise erfolgende Hydrierung von D-Fructose zu Mannit ist an pulverförmigen kupferhaltigen Gerüstkatalysatoren und trägergebundenen Kupferkatalysatoren für den Einsatz von 20%igen wäßrigen Fructose-Lösungen bei Temperaturen von 50-60°C und einem Wasserstoffdruck von 50-60bar in der Literatur beschrieben. Für unterschiedlich modifizierte (z.B. mit Co, Fe, B, Zn oder Cr dotierte) Raney-Kupfer-Katalysatoren liegen die Selektivitäten zu Mannit bei 60-65%. Mit den Raney-Katalysatoren werden Umsätze von bis zu 97% erreicht [M. Hegedüs, S. Göbölös, J.L. Margitfalvi in "Heterogeneous Catalysis and Fine Chemicals III", M. Guisnet et al. (Ed.), 1993 Elsevier Publishers, 187-194; HU 60230].

In der DE 197 20 496 wird ein Verfahren zur Hydrierung von Zuckern oder Zuckergemischen zu Zuckeralkoholen oder Zuckeralkoholgemischen beschrieben, wobei die Zucker oder Zuckergemische in wäßriger Lösung bei erhöhter Temperatur und erhöhtem Druck mit Wasserstoff unter Verwendung eines ein Gemisch aus einem reinen Raney-Metall und einer Raney-Metallegierung enthaltenden Schalenkatalysators hydriert werden und wobei der Schalenkatalysator einen katalytisch weitgehend inaktiven und als Träger wirkenden Kern und eine katalytisch aktive Schale aufweist. In einem diskontinuierlichen Verfahren wird 30 Gew.-%ige wäßrige Fructoselösung unter Verwendung eines Katalysators in Tablettenform bestehend aus einer Kupfer/Aluminium-Legierung (Cu : Al gleich 50 : 50 Gew.-%) und reinem Kupfer als Binder im Gewichtsverhältnis 100 : 15 bei 90 °C und einer Reaktionszeit von 22 h hydriert und ein Umsatz von 98,4% erzielt. Es werden 61,6 Gew.-% Mannit, 36,2 % Sorbit, 0,12 Gew.-% Glucose und 0,52 Gew.-% weitere Nebenprodukte gebildet.

Aufgabe der vorliegenden Erfindung war es, ein Verfahren zur Herstellung von Mannit aus Fructose zu entwickeln, welches die Nachteile bekannter Verfahren vermeidet oder zumindest vermindert und insbesondere bei kurzer Reaktionszeit die Herstellung von Mannit aus Fructose mit hohen Umsätzen und unter geringer Nebenproduktbildung ermöglicht.

Überraschenderweise wurde nun gefunden, daß diese Aufgabe gelöst wird, wenn das Verfahren zur Herstellung von Mannit aus Fructose so durchgeführt wird, daß die Hydrierung von Fructose in Gegenwart eines Raney-Kupfer Katalysators erfolgt und in kontinuierlicher Fahrweise durchgeführt wird.

Die Erfindung betriff somit ein Verfahren zur Herstellung von Mannit durch kontinuierliche Hydrierung von Fructose in wässriger Lösung in einem Reaktor mit einer Katalysatorschüttung, bestehend aus einem Raney-Kupfer-Katalysator, welcher erhältlich ist, durch Mahlen und Sieben einer Kupfer-Aluminiumlegierung und durch Aktivierung der Oberfläche der erhaltenen Partikel mit einer 10 - 20 %igen Natronlauge bei 20 - 80°C.

Das erfindungsgemäße Verfahren zeichnet sich insbesondere dadurch aus, daß die Herstellung von Mannit aus Fructose bei kurzen Reaktionszeiten mit hohen Umsätzen und unter geringer Nebenproduktbildung erfolgt.

Die vorliegende Erfindung stellt ein vorteilhaftes Verfahren zur Herstellung von Mannit durch Reduktion bzw. Hydrierung von Fructose zur Verfügung. Nach diesem Verfahren wird eine wäßrige Fructoselösung über ein Festbett aus einem Raney-Kupfer Katalysator geleitet.

Die Fructose hat eine Reinheit von 90-100%, vorzugsweise von 95-100%. Hauptverunreinigung hierbei ist Glucose.

Die für das erfindungsgemäße Verfahren geeigneten Raney-Kupfer Katalysatoren sind käuflich erwerbbar oder können nach bekannten Methoden hergestellt werden [s. z.B. M. Hegedüs, S. Göbölös, J.L. Margitfalvi in "Heterogeneous Catalysis and Fine Chemicals III", M. Guisnet et al. (Ed.), 1993 Elsevier Publishers, 187-194; HU 60230]. Zur Herstellung der Raney-Kupfer Katalysatoren wird eine Kupfer-Aluminium-Legierung, in die gewünschte Partikelgröße gemahlen und gesiebt, und durch Behandlung mit 10-20%iger wäßriger Natronlauge bei 20-80°C auf der Oberfläche aktiviert. Hierbei bildet sich ein Schalenkatalysator aus, der aus einem inaktiven Kern aus Raney-Legierung und einer aktiven Oberfläche aus dem entsprechenden Raney-Metall besteht. Die Schalendicke ist abhängig von der Aktivierungsdauer der Katalysatorpartikel.

Zur Herstellung der für das erfindungsgemäße Verfahren verwendeten Katalysatoren werden keine Bindemittel für die Raney-Kupferlegierung, wie beispielsweise reines Raney-Metall, verwendet.

Die für das erfindungsgemäße Verfahren verwendeten Katalysatoren besitzen den Vorteil, daß sie sehr kostengünstig und einfach herstellbar sind. Zudem müssen sie für die eigentliche Reaktion nicht durch Vorreduktion aktiviert werden und besitzen unter Wasser eine hohe Lagerstabilität.

Die für das erfindungsgemäße Verfahren verwendeten Katalysatoren können dotiert sein, d.h. die Kupfer-Aluminium-Legierung kann insgesamt 0,1-20 Gew.-% an anderen Elementen enthalten, wie z.B. Bor, Chrom, Cobalt, Eisen, Molybdän, Titan oder Zink.

Die Reaktion kann z.B. in einem Rieselbettreaktor stattfinden. Hierbei ist ein senkrecht stehender Rohrreaktor mit der Katalysatorschüttung gefüllt und wird von der wäßrigen Zuckerlösung im Gleichstrom mit dem Wasserstoff durchrieselt bzw. durchströmt. In einem alternativen Verfahren kann die Katalysatorschüttung beispielsweise in einer Art Blasensäule von der wäßrigen Zuckerlösung im Gleichstrom mit dem Wasserstoff von unten durchströmt werden.

In einer bevorzugten Verfahrensvariante wird über mehrere hintereinander geschaltete Reaktoren, beispielsweise vier hintereinandergeschaltete Reaktoren, ein Temperaturgradient angelegt. Bei dieser Verfahrensvariante wird wiederum vorteilhaft in dem ersten Reaktor bei relativ niedrigen Anfangstemperaturen hydriert und in den folgenden Reaktoren bei höheren Temperaturen der Restzucker hydriert. Hierdurch kann der Anteil an Nebenprodukten bei einem Umsatz größer 99,8 % unter 1 Gew.-% gehalten werden.

Geeignete Reaktionstemperaturen für das erfindungsgemäße Verfahren sind Temperaturen zwischen 50 und 180°C. Vorzugsweise wird das erfindungsgemäße Verfahren bei Reaktionstemperaturen von 90 bis 140°C durchgeführt.

Geeignete Wasserstoffdrücke für das erfindungsgemäße Verfahren sind Wasserstoffdrücke zwischen 50 und 300 bar. Vorzugsweise wird das erfindungsgemäße Verfahren bei Wasserstoffdrücken von 150 bis 250 bar durchgeführt. Insbesondere bevorzugt wird das erfindungsgemäße Verfahren bei Wasserstoffdrücken von 160 bis 200 bar durchgeführt.

Geeignete Konzentrationen von Fructose in Wasser für das erfindungsgemäße Verfahren sind Konzentrationen von 10 bis 70 Gew.-%. Vorzugsweise wird das erfindungsgemäße Verfahren mit Konzentrationen von Fructose in Wasser von 40 bis 60 Gew.-% durchgeführt. Insbesondere bevorzugt wird das erfindungsgemäße Verfahren mit Konzentrationen von Fructose in Wasser von 50 bis 55 Gew.-% durchgeführt.

Die hohen Konzentrationen der wäßrigen Eduktlösung, die bei Anwendung des erfindungsgemäßen Verfahrens realisiert werden können, haben den Vorteil, daß zur Aufarbeitung der Produktlösung nur wenig Wasser entfernt werden muß. Hierdurch kann z.B. der Energieverbrauch im Vergleich zu Verfahren, die niedrigere Eduktkonzentrationen verwenden, signifikant abgesenkt werden.

Geeignete Verhältnisse von Fructose zu Wasserstoff für das erfindungsgemäße Verfahren sind Verhältnisse von 1mol Fructose : 1mol Wasserstoff bis 1mol Fructose : 100mol Wasserstoff. Vorzugsweise wird das erfindungsgemäße Verfahren mit Verhältnissen von Fructose zu Wasserstoff von 1mol Fructose: 5mol Wasserstoff bis 1mol Fructose : 40mol Wasserstoff durchgeführt.

Eine geeignete Reaktorbelastung (Verhältnis Volumenstrom Zuckerlösung zu Katalysatorvolumen) für das erfindungsgemäße Verfahren liegt bei LHSV 0,01 h⁻¹ bis 10,0 h⁻¹. Vorzugsweise wird das erfindungsgemäße Verfahren mit LHSV 0,1 h⁻¹ bis 1,0 h⁻¹ durchgeführt.

Vorzugsweise wird das erfindungsgemäße Verfahren bei einem pH-Wert von 3,0 bis 12,0 durchgeführt, insbesondere bei einem pH-Wert von 4,0 bis 6,0.

Das Fortschreiten bzw. das Ende der Reaktion sowie die Analyse der Reaktionsprodukte kann z.B. mittels HPLC erfolgen, z.B. unter Verwendung von Standard-HPLC-Geräten mit Kalzium-Ionentauscher-Säulen.

Nach beendeter Reaktion erfolgt die Isolierung des Reaktionsprodukts nach gängigen Methoden. Unter "üblicher Aufarbeitung" wird im Rahmen der vorliegenden Erfindung folgendes verstanden:

Das Reaktionsgemisch wird in einem Hochdruckabscheider aufgefangen und der Wasserstoff nach Ersatz des verbrauchten Wasserstoffs recycelt. Die Lösung wird anschließend entspannt, wobei sie üblicherweise eine Temperatur von ca. 90-95 °C besitzt. Sie wird zur Entfernung von Katalysatorresten heiß filtriert (bei Temperaturen größer 60° C) und danach zur Abtrennung von ionischen Verunreinigungen über Anionenund Kationentauscherharze gereinigt.

Die Gewinnung von Mannit aus der aufgereinigten Hydrierlösung kann nun durch Kristallisation des Mannits aus der Mutterlauge oder durch chromatographische Trennung der beiden Hauptprodukte Mannit und Sorbit erfolgen.

Auch ohne weitere Ausführungen wird davon ausgegangen, daß ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen.

Die folgenden Beispiele sollen die vorliegende Erfindung verdeutlichen. Sie sind jedoch keinesfalls als limitierend zu betrachten.

### Beispiele

Es wird ein handelsüblicher Raney-Kupfer-Festbettkatalysator (Al: 39,0%; Cu: 61,0%) verwendet. Die Partikelgröße der unregelmäßig geformten Teilchen liegt bei ca. 2x3mm. Die Katalysatorschüttung weist ein Freivolumen von ca. 50% auf, die Schüttdichte liegt bei ca. 1,75 g/cm³.

Die Reaktionskontrolle und die Analyse der Reaktionsprodukte erfolgt mittels HPLC.

### Beispiel 1

In einem Hydrierreaktor mit 120 ml Volumen wird im kontinuierlichen Festbettverfahren eine Fructoselösung hydriert. Der Rohrreaktor besteht aus einem senkrecht stehenden Edelstahlrohr mit 25cm Länge und 2,5cm Innendurchmesser. Am Boden des Reaktors befindet sich eine Metallfritte, der Einlaß von Hydrierlösung und Wasserstoff erfolgt im Gleichstrom von oben (Rieselbettreaktor).

Im Rohrreaktor befinden sich 241,5g (feucht) Raney-Kupfer-Katalysator. Bei einem Rohrreaktorvolumen von 120ml wird ein Fluß von 60ml/h (LHSV=0,5 h⁻¹) an Fructoselösung (50 Gew.-%ige wäßrige Lösung) angelegt. Der Druck liegt bei 170bar und die Temperatur bei 110°C. Es wird ein Wasserstoffstrom von 75NI/h angelegt. Die Reinheit der verwendeten Fructose liegt bei 100%. Die Ergebnisse der HPLC-Analytik sind in Tabelle 1 dargestellt.

### Beispiel 2

Die Reaktion wird wie in Beispiel 1 beschrieben durchgeführt. Anstelle einer reinen Fructoselösung wird jedoch ein Edukt, das zu 96,0 Gew.-% aus Fructose besteht, verwendet. Die Ergebnisse der HPLC-Analytik sind in Tabelle 1 dargestellt.

**Tabelle 1**

| | Beispiel 1 | | Beispiel 2 | |
|---|---|---|---|---|
| Komponente | Edukt [Gew.-%] | Produkt [Gew.-%] | Edukt [Gew.-%] | Produkt [Gew.-%] |
| Fructose | 100,0 | 1,4 | 96,0 | 5,4 |
| Glucose | - | 0,1 | 3,7 | 0,8 |
| Saccharose | - | - | 0,3 | 0,1 |
| Mannit | - | 62,8 | - | 57,9 |
| Sorbit | - | 35,4 | - | 35,5 |
| Weitere Nebenprodukte | - | 0,3 | - | 0,3 |

Das Verhältnis von Mannit zu Sorbit verringert sich in Beispiel 2 dadurch, daß der Glucose-Anteil des Edukts zu Sorbit hydriert wird.

## Patentansprüche

1. Verfahren zur Herstellung von Mannit durch kontinuierliche Hydrierung von Fructose in wässriger Lösung in einem Reaktor mit einer Katalysatorschüttung, bestehend aus einem Raney-Kupfer-Katalysator, welcher erhältlich ist, durch Mahlen und Sieben einer Kupfer-Aluminiumlegierung und durch Aktivierung der Oberfläche der erhaltenen Partikel mit einer 10 - 20 %igen Natronlauge bei 20 - 80 °C.

2. Verfahren gemäß Anspruch 1, **dadurch gekennzeichnet, dass** die Kupfer-Aluminiumlegierung durch 0,1-20 Gew.-% eines oder mehrerer Element(s)e ausgewählt aus der Gruppe Bor, Chrom, Cobalt, Eisen, Molybdän, Titan und Zink dotiert sein kann.

3. Verfahren gemäß Anspruch 1, **dadurch gekennzeichnet, dass** eine Kupfer-Aluminiumlegierung mit einem Kupfer-Aluminium-Verhältnis von 61 : 39 % verwendet wird.

4. Verfahren gemäß Anspruch 1, **dadurch gekennzeichnet, dass** eine Kupfer-Afuminiumlegierung in Form unregelmäßig geformter Teilchen mit einer Partikelgröße von ca. 2 x 3 mm als Katalysator verwendet wird, welcher in der Katalysatorschüttung ein Freivolumen von ca. 50 % und eine Schüttdichte von ca. 1, 75 g/cm³ aufweist.

5. Verfahren gemäß einem oder mehreren der Ansprüche 1 - 4, **dadurch gekennzeichnet, dass** die Reaktion bei einer Reaktionstemperatur von 50 bis 180 °C durchgeführt wird und bei einem Wasserstoffdruck von 50 bis 300 bar durchgeführt wird.

6. Verfahren gemäß einem oder mehreren der Ansprüche 1-4, **dadurch gekennzeichnet, dass** die Reaktion bei einer Reaktionstemperatur von 110 °C und einem Wasserstoffdruck von 170 bar durchgeführt wird.

7. Verfahren gemäß einem oder mehreren der Ansprüche 1-4, **dadurch gekennzeichnet, dass** die Konzentration von Fructose in Wasser 40 bis 60 Gew.-%, vorzugsweise 50-55 Gew.-% beträgt.

8. Verfahren gemäß einem oder mehreren der Ansprüche 1 bis 7 **dadurch gekennzeichnet, dass** die Reaktorbelastung bei LHSV 0,01 h⁻¹ bis 10,0 h⁻¹ liegt, vorzugsweise bei 0,1 h⁻¹ bis 1,0 h⁻¹.

## Claims

1. Process for preparing mannitol by continuous hydrogenation of fructose in aqueous solution in a reactor with a catalyst bed consisting of a Raney copper catalyst which is obtainable by grinding and screening a copper/aluminium alloy and by activating the surface of the resulting particles with a 10-20% sodium hydroxide solution at 20-80°C.

2. Process according to Claim 1, **characterized in that** the copper/aluminium alloy may be doped by 0.1-20% by weight of one or more element(s) selected from the group of boron, chromium, cobalt, iron, molybdenum, titanium and zinc.

3. Process according to Claim 1, **characterized in that** a copper/aluminium alloy with a copper/aluminium ratio of 61:39% is used.

4. Process according to Claim 1, **characterized in that** a copper/aluminium alloy in the form of irregularly shaped particles with a particle size of about 2x3 mm is used as catalyst, which has a free volume of about 50% and an apparent density of about 1.75 g/cm³ in the catalyst bed.

5. Process according to one or more of Claims 1-4, **characterized in that** the reaction is carried out at a reaction temperature of from 50 to 180°C and with a hydrogen pressure of from 50 to 300 bar.

6. Process according to one or more of Claims 1-4, **characterized in that** the reaction is carried out at a reaction temperature of 110°C and a hydrogen pressure of 170 bar.

7. Process according to one or more of Claims 1-4, **characterized in that** the concentration of fructose in water is from 40 to 60% by weight, preferably from 50-55% by weight.

8. Process according to one or more of Claims 1 to 7, **characterized in that** the space velocity is an LHSV of from 0.01 h⁻¹ to 10.0 h⁻¹ preferably 0.1 h⁻¹ to 1.0 h⁻¹.

## Revendications

1. Procédé pour la préparation de mannite par une hydrogénation continue de fructose dans une solution aqueuse et dans un réacteur avec un débit de catalyseur, constitué d'un catalyseur de cuivre de Raney, lequel peut être obtenu en moulant et en tamisant un alliage de cuivre-aluminium et en activant la surface des particules obtenues à l'aide d'une soude caustique à 10-20 % à 20-80°C.

2. Procédé selon la revendication 1, **caractérisé en ce que** l'alliage cuivre-aluminium peut être dopé par 0,1-20 % en poids d'un ou de plusieurs élément(s) choisi dans le groupe comprenant du bore, du chrome, du cobalt, du fer, du molybdène, du titane et du zinc.

3. Procédé selon la revendication 1, **caractérisé en ce qu'**un alliage de cuivre-aluminium est utilisé dans une proportion cuivre-aluminium de 61 : 39 %.

4. Procédé selon la revendication 1, **caractérisé en ce qu'**un alliage cuivre-aluminium est utilisé comme catalyseur sous la forme de particules irrégulières d'une taille particulaire d'environ 2 x 3 mn, lequel présente dans le débit de catalyseur un volume libre d'environ 50 % et une densité en vrac d'environ 1,75 g/cm³.

5. Procédé selon une ou plusieurs des revendications 1 à 4, **caractérisé en ce que** la réaction est réalisée à une température de réaction allant de 50 à 180°C et à une pression d'hydrogène allant de 50 à 300 bars.

6. Procédé selon une ou plusieurs des revendications 1 à 4, **caractérisé en ce que** la réaction est réalisée à une température de réaction de 110°C et à une pression d'hydrogène de 170 bars.

7. Procédé selon une ou plusieurs des revendications 1 à 4, **caractérisé en ce que** la concentration de fructose dans l'eau va de 40 à 60 % en poids, de préférence de 50 à 55 % en poids.

8. Procédé selon une ou plusieurs des revendications 1 à 7, **caractérisé en ce que** la charge du réacteur va de 0,01 h⁻¹ à 10,0 h⁻¹ en vitesse spatiale horaire du liquide, de préférence de 0,1 h⁻¹ à 1,0 h⁻¹.
